# EUROPEAN PATENT SPECIFICATION

(11) **EP 2 217 204 B1**
(45) Date of publication and mention of the grant of the patent: **28.06.2017**
(21) Application number: 08847751.8
(22) Date of filing: 05.11.2008
(51) Int. Cl.: A61K 9/00, A61K 31/47, A61K 31/728, A61K 47/34, A61K 47/36

(54) **COMPOSITIONS FOR THE TOPICAL PROTECTION OF THE OCULAR TISSUES FROM THE DAMAGING EFFECTS OF ULTRAVIOLET RADIATIONS**
ZUSAMMENSETZUNGEN ZUM TOPISCHEN SCHUTZ DES OKULARGEWEBES VOR DEN SCHÄDLICHEN EFFEKTEN VON ULTRAVIOLETTSTRAHLUNGEN
COMPOSITIONS POUR LA PROTECTION TOPIQUE DES TISSUS OCULAIRES À PARTIR DES EFFETS NUISIBLES DES RAYONNEMENTS À ULTRAVIOLETS

(30) Priority: 07.11.2007 EP 07425699
(43) Date of publication of application: 18.08.2010
(73) Proprietor: Bruschettini S.r.l., 16147 Genova (IT)
(72) Inventor: BOZZO COSTA, Edoardo, 16136 Genova (IT)
(74) Representative: Minoja, Fabrizio
(86) International application number: PCT/EP2008/009309
(87) International publication number: WO 2009/059748

(56) References cited:
- WO-A-2006/120696
- CIURLO G ET AL: "Richerche sperimentali sull'azione protettiva di un preparato chinolinico contro gli effetti nocivi dei raggi u.v. sui tessuti oculari" ANNALI DI OTTALMOLOGIE E CLINICA OCULISTICA, CEM CASA EDITRICE MACCARI, PARMA, IT, vol. 91, June 1995 (1995-06), pages 394-400, XP009098679 ISSN: 0003-4665 cited in the application

## Description

The present invention relates to topical compositions for the protection of the ocular tissues from the damaging effects of ultraviolet radiations.

### BACKGROUND OF THE INVENTION

The human eye may be subjected to stress from non ionizing radiations, particularly UV radiations, under particular environmental conditions, either natural or artificial. UV radiations may produce only functional consequences (dazzling, burning, irritation) in case of medium intensity, short exposition times, but may cause serious damaging alterations of organic nature on the retina, cornea, crystalline lens and conjunctiva in case of high intensity, prolonged exposition.

The main source of UV radiations is the sun light whose damaging action on the eye can be enhanced by reflecting surfaces as water, snow, sand. Scientific studies reported data concerning the extremely serious dangerousness of short wave UV radiation on the ocular tissues.

Artificial sources of UV radiations (electric arc welding, suntan light, artificial UV lamps for cosmetic use, bactericidal lamps for sanitary use), as well as a number of diagnostic tests in ophthalmoscopy may also cause damaging effects on the ocular tissues.

At present, the only protection device for the eyes are the sunglasses with a protective screen against damaging ultraviolet radiations. However, it is very difficult for subjects exposed for a long time to UV radiations to continuously wear sunglasses.

A special effectiveness against UV radiations is ascribed to quinoline derivatives: hydroxyquinoline methyl sulphate and n-methylbenzoquinoline methyl sulphate.

Hydroxyquinoline methyl sulphate has, however, some disadvantages: a yellow colour unsuited for soft contact lenses wearers as it is absorbed by the lenses polymeric structure, and very low mucoadhesivity on the ocular surfaces.

n-Methylbenzoquinoline methyl sulphate (mebequinium methyl sulphate D.C. IT.) is a known compound. Said quinoline derivative is an excellent UV screen, absorbing UV radiations of wavelengths between 280 and 315 nm, which are the most dangerous to the ocular tissues (Ricci G. Ann. Oftalmol Clin. Oculist., 86, 11,1960). This compound is present in Ascotodin®, an eye drop solution with specific antihistaminic indication, developed and marketed by the Applicant, containing an association of thonzylamine hydrochloride (antihistaminic drug), n-methylbenzoquinoline methyl sulphate (as a UV screen) and carboxymethylcellulose (as a viscosizing agent).

Studies performed in animals (rabbit) (Ciurlo G. Altieri G. Ann. Oftalm. Clin. Oculist., 91,394,1965; Pescosolido N. et al. Atti Fond. G. Ronchi 47.6, 933-42.1992) and several clinical trials (Salducci M.Carelli M. Ann. Oft. Clin. Oculist. 117,1,1-5,1991; Orione C. Boll. Oculist. 72,1,33-39,1993; Gugliermina P. et al. Boll. Oculist., 72, 1,21-32,1993; Fiorani M. et al. Ann. Oft. Clin. Oculist. 116,85,1990) proved that Ascotodin® is an effective UV screen and has good tolerability.

This ophthalmic preparation has however two important limits:
1) the presence of thonzylamine HCl, an antihistaminic substance, prevents long-lasting use, due to its well known pharmacological side effects (rash, urticaria, angioedema, photosensitization, ocular hypertension);
2) carboxymethylcellulose is contained in a low percentage that does not assure the long-lasting mucoadhesion of the UV screen on the corneal surface.

In view of the above reasons, there is the need for a highly effective topical protection for the ocular tissues (retina, lens and cornea above all) which also provides better individual tolerability.

### BRIEF SUMMARY OF THE INVENTION

It has now been found that a combination of n-methylbenzoquinoline methyl sulphate, glycerin, hyaluronic acid and PVA effectively protects the ocular tissues from the damaging effects of ultraviolet radiations.

### DISCLOSURE OF THE INVENTION

It has unexpectedly been found that n-methylbenzoquinoline methyl sulphate, a UV screening compound, suitably formulated in the form of a colloidal solution, a micro emulsion, or a viscous hydrogel, can protect ocular tissues from the damaging effects of UV radiations, particularly of wavelength between 280 and 315 nm. Such screening effect is long-lasting thanks to the presence of a carrier comprising polyvinyl alcohol (PVA), sodium hyaluronate and glycerin in specific concentrations.

The invention, therefore, relates to an ophthalmic composition comprising:
a) n-methylbenzoquinoline methyl sulphate (MBQM) or its salts;
b) polyvinyl alcohol;
c) sodium hyaluronate; and
d) glycerin;
for the protection of the ocular tissues from the damaging effects of ultraviolet radiations.

n-Methylbenzoquinoline methyl sulphate (MBQM) is an effective UV screen, well tolerated and free from any pharmacological or mutagenic activities, therefore it may be used even for long-term treatments. Highly pure MBQM for the ophthalmic use is employed, having heavy metals content lower than 10 ppm. MBQM is dissolved in an aqueous colloidal isotonic hydrogel.

According to preferred aspect of the invention, MBQM or its salts will be present in amounts ranging from 0.1% to 1% by weight, more preferably 0.2% to 0.4%, based on the total weight of the composition.

Polyvinyl Alcohol (PVA) is a non ionic synthetic polymer endowed with mucomimetic properties, such as soothing and lubricating actions on the eye surfaces. Its low surface tension enhances the stability of the tear film thereby increasing cornea wetting and reduces the surface tension of the tears. The very thin film formed by PVA on the cornea and conjunctiva surfaces provides the protection of epithelium, and is essential to assure the long-lasting contact of the MBQM UV screen on these tissues.

PVA will be present in the ophthalmic composition of the invention in an amount effective to provide the prolonged contact of the hydrogel containing the active UV screen with the cornea and other ocular tissues upon topical administration on the eye, thereby ensuring the long term protection from UV damaging radiations. According to a preferred aspect of the invention, PVA will be present in amounts ranging from 0.8% to 1.6% by weight, preferably from 1.0 to 1.4%, on the total weight of the composition.

Sodium hyaluronate is a safe, well tolerated colloidal polymer present in human tears, vitreous body and aqueous humour of the eye, with stabilizing and lubricant action. When combined with PVA in a specific ratio, it exerts a synergistic action, giving rise to a very thin, stable viscohelastic hydrogel which increases the long term contact of the MBQM UV screen on the ocular surfaces. According to a preferred aspect of the invention, sodium hyaluronate will be present in amounts ranging from 0.005% to 0.1% by weight of the composition.

Glycerin is useful to increase viscoelasticity and hydrophilicity of the hydrogel and to prevent the excessive corneal hydration (oedema) and consequent ocular irritation. According to a preferred aspect of the invention, glycerin will be present in amounts ranging from 0.3 to 1.5% by weight of the composition.

The pH of the ophthalmic solution of the present invention should range between 6.0 and 7.0, preferably between 6.4 and 6.8. A buffer system can be added to the above components in order to maintain the pH at the requested values. A suitable buffer can be the combination of monobasic and dibasic sodium and/or potassium phosphate, but other buffer systems can be used (sodium tetraborate-boric acid, bicarbonate, citrate).

The formulations of the invention can also contain osmolarity agents, such as sodium chloride, to provide iso-osmolarity of the colloidal solution with the lachrymal liquid.

The formulations of the invention can further comprise the preservatives conventionally used in eye drop solutions, as long as they do not interfere with the MBQM UV screening effect and they are not adsorbed on the polymeric structure of soft contact lenses. Preservatives must be safe and well tolerated even for long term treatments. Examples of suitable preservatives are methyl and propyl p-hydroxybenzoates in low concentrations, possibly in combination with disodium edetate, a chelating agent which enhances the bactericide effects of parabens in the composition. Other suitable preservatives include hexamethylene biguanide, benzyl and phenyl ethyl alcohols.

The formulations of the invention can further comprise Hamamelis distilled water for its well known demulcent and soothing effects.

The formulations of the invention can further comprise conventional additives as long as they do not inhibit the UV absorbing activity of the screening substance neither modify the stability of the colloidal hydrogel.

The compositions of the present invention provide a long lasting screening action against UV damaging radiations within the range of 280 - 315 nm. This enduring effect is due to the viscohelastic hydrogel, which maintains the UV screening substance incorporated in the network of the thin colloidal film adhering to the ocular mucosal tissues for long-time periods (over 3 hours).

The aqueous colloidal solution of the present invention can be instilled into the eye conjunctival lower sac in order to protect ocular tissues from ultraviolet radiations. Indeed, due to the presence of MBQM, the colloidal hydrogel absorbs in high percentage the UV radiations of wavelength among 280 and 315 nm, that are the most dangerous to cornea and retina.

Without intending to be bound to any theory, the inventor believes that MBQM (mebequinium methyl sulphate DC.IT.), when incorporated in the colloidal viscohelastic hydrogel described above, forms a very thin and stable colloidal mucomimetic, viscohelastic film protecting for some hours the ocular tissues from the UV radiations damaging effects.

According to the invention, one or two drops of the compositions of the invention will be instilled into the lower conjunctival sac of each eye every 2-3 hours or more frequently, if requested.

The compositions of the present invention can be used both in humans and animals, such as but not limited to canines, cats, and equines.

The compositions of the invention may be formulated in the form of a moderately viscous colloidal solution, a micro emulsion, or a viscous hydrogel.

A suitable formulation advantageously contains the following ingredients in the following percentages by weight:

| | |
|---|---|
| n-Methylbenzoquinoline methyl sulphate | 0.300% |
| Polyvinyl alcohol | 1.250% |
| Sodium hyaluronate | 0.010% |
| Glycerin | 0.800% |
| Hamamelis distilled water | 0.100% |
| Dibasic sodium phosphate | 0.090% |
| Monobasic potassium phosphate | 0.020% |
| Disodium edetate | 0.030% |
| Methyl-p-hydroxy benzoate | 0.030% |
| Propyl-p-hydroxy benzoate | 0.012% |
| Sodium chloride | 0.600% |
| Purified water | q.s. to ml 100 |

| | |
|---|---|
| Note: the pH of the solution should be within the range of 6.0-7.0, preferably pH 6,4-6,8. | |

### FORMULATION EXAMPLES

**FORMULATION n. 1: Aqueous colloidal ophthalmic composition**

| Ingredient | Amount in grams |
|---|---|
| Purified water | 980.00 |
| n-Methylbenzoquinoline methyl sulphate | 3.00 |
| Polyvinyl alcohol | 12.50 |
| Sodium hyaluronate | 0.10 |
| Glycerin | 8.00 |
| Hamamelis distilled water | 1.00 |
| Disodium edetate | 0.30 |
| Dibasic sodium phosphate 12 H₂O | 0.90* |
| Monobasic potassium phosphate | 0.20* |
| Sodium chloride | 6.00 |
| Methyl-p-hydroxybenzoate | 0.30 |
| Propyl-p-hydroxybenzoate | 0.12 |
| Total | 1010.92 |

| | |
|---|---|
| * or the amount necessary to adjust the pH to 6.0-6.8 | |

**Formulation n. 2: Aqueous colloidal ophthalmic composition**

| Ingredient | Amount in grams |
|---|---|
| Purified water | 970.00 |
| n-Methylbenzoquinoline methyl sulphate | 3.00 |
| Polyvinyl alcohol | 12.50 |
| Sodium hyaluronate | 0.10 |
| Glycerin | 8.00 |
| Hamamelis distilled water | 1.00 |
| Disodium edetate | 0.30 |
| Sodium tetraborate | 3.00* |
| Boric acid | 15.00* |
| Polyhexamethylene biguanide | 0.001 |
| Sodium chloride | 1.00 |
| Total | 1012.701 |

| | |
|---|---|
| * or the amount necessary to adjust the pH to 6.0-6,8 | |

The compositions of the invention were tested for their tensiometric surface properties at the CNR-IENI Research National Council Institute for Energetic and Interphases in Genoa, Italy.

The results of such experimentation are reported in the following.

The characterisation of tensiometric surface properties of samples of some illustrative formulations disclosed above was carried out as follows.

Samples of UV screen eye drops with two different concentrations of sodium hyaluronate (0.010% and 0.100%) were used.

Measurements were carried out according to bubble profile tensiometry and capillary pressure tensiometry, using a PAT (Sinterface - Germany) and CPT (CNR - Genoa -Italy) instrumentations, respectively.

Temperature measurements: 20°C.

The stability of a liquid film laid on a solid surface, in the specific case on the ocular surface, depends on many factors, the most important of which being the affinity of the liquid to the surface which it is laid on, or otherwise its adhesion properties. Other factors are the viscous characteristics of the colloidal liquid and the properties of the liquid-air surface.

The present study assayed the tensiometric surface properties (expressed as gamma) which play an important role as they are an indication of the film response to the alterations of the surface area.

On the other hand, the rheological superficial dilatational properties, expressed as the module of dilatational viscoelasticity defined as epsilon = delta gamma/delta Ina and therefore stating the answer of surface tension to variation of area "a", are very important.

The aim of the study was to evaluate whether the variation of the concentration of sodium hyaluronate in the solution of the present invention, involves a significant variation of tensiometric properties of liquid/air surface and therefore can affect the stability of the film laid on the ocular surface.

### Results

From the evolution of surface tension, the tested samples proved to contain soluble tensioactive components piling upon the liquid/air surface.

The data show that two samples, corresponding to 1.0 and 0.1 grams per litre of sodium hyaluronate, have very similar characteristics and a value of surface tension of balance almost identical resulting to be gamma = 42,2 plus-minus 0.1 mn/m for both samples.

The module of surface viscoelasticity "epsilon" was evaluated with the method of the swinging (oscillating) drop, as a function of the frequency "v" of surface area perturbation.

The "PAT" bubble profile tensiometer and the CPT tensiometer for this test has been used.

The surface rheological properties and the dilatational surface viscosity values were very similar for both samples and they corresponded to a limit elasticity value at high frequency of 12.8 mn/m and to a characteristic frequency of 0.2 Hz. These values are near to those of tensioactive systems used for stabilizing liquid colloidal films (useful in colloidal eye drop solutions).

An experimental study on rheological properties of artificial tears (tears substitutes) (Bother H. et al Drug development and industrial Pharmacy. 16.1990-755-768) viscosity of these colloidal solutions is tested as a function of the shear rate. As human tears liquids are physiologically stressed by alterations such as "shear" as a consequence of eyelids blinking, suitable artificial tear substitutes should have a non-newtonian behaviour (high "zero shear viscosity" and highly decreasing viscosity with increasing frequency of shears). The study performed by CNR IENI of Genoa highlights the more specifically superficial side of the rheology of the tested solutions of the present inventions.

Actually during blinking the ocular surface is subjected to local fluctuations of superficial area.

### Conclusions

The two sodium hyaluronate concentrations (0.1% - 1.0%) in the tested samples were equivalent as far as the surface tension value and the dilatational rheological properties are concerned.

The experimental data obtained in this study show that both tested colloidal formulation of the invention were effective in terms of surface liquid/air properties and rheological characteristics.

A dilatational viscosity decreasing with frequency, as observed in the tested compositions of the invention (Figure), can be considered a suitable characteristic for a tear substitute.

### Tests on rabbit eyes

Protection of the ocular tissues from UV damaging effects was tested in rabbit ocular tissues. One of the illustrative formulations of the invention (Formulation No. 1, in the following referred to as Bruviscreen) was compared with the commercial formulation Ascotodin® having the following formulation:

| | |
|---|---|
| n-methyl-benzoquinoline methyl sulphate | 0.300 grams |
| thonzylamine hydrochloride | 0.100 |
| sodium tetraborate decahydrate | 0.023 |
| sodium chloride | 0.900 |
| carboxymethylcellulose | 0.033 |
| parahydroxybenzoate esters | 0.047 |
| purified water | up to ml 100. |

The Formulation No. 1 of the present invention and Ascotodin® were topically applied to the right eye of the rabbits while corresponding controls consisting of a sodium chloride 0.9% aqueous solution were topically applied to the left eye.

Experimental procedures were conducted substantially as described by Caramazza N. et al. in the study issued on Boll. oculist., 72,3-19, 1993.

Four albino rabbits (male) weighing 3.000-3500 g were used.

Animal experiments were conform to the guidelines of the ARVO resolution on the use of animals in research.

Two animals were treated with Formulation No. 1, two other animals with Ascotodin®. The right eyes of each rabbit were instilled with two drops of Formulation No. 1 or Ascotodin®, respectively, while the left eyes of the animals were similarly instilled with saline (0.9% sodium chloride solution), three times/day during three consecutive days.

The fourth day the eyes of the rabbits were instilled for the last time and immediately after the eyes were exposed to a light of an ultraviolet (UV 280 nm) lamp of 50 w potency at a length of 65 cm during 28 minutes.

### Observations

24 hours after the UV damaging radiations exposition, all the eyes of the rabbits were examined macroscopically with an ophthalmoscope, and, after killing animals, an histological examination of the ocular tissues was performed.

Surprisingly, in Bruviscreen treated rabbits the corneal tissues resulted protected from the UV damaging radiations, in fact histological examination revealed only focal hyperplasia of epithelium, absence of phlogosis and very modest vascular hyperhemia with modest hematic congestion, absence of degenerative phenomena, very low ectasia of the vessels with modest hematic congestion.

Unexpectedly, histological examination of Ascotodin® treated rabbits showed that MBQM at the same concentration as in Bruviscreen, but vehicled in a hydrogel only consisting of carboxymethylcellulose in low concentration, was ineffective in protecting the eyes of rabbits from UV radiation. A number of evident damaging effects were observed in the cornea: severe and irregular epithelium hyperplasia with focal and diffused erosion; severe mixtocellular phlogosis of superficial, medium and deep layers of connectival stroma of mucosa, including heterophils, plasma cells, lymphocytes.

Plasma cells are actively going through the epithelial cells of the basal layer of the same epithelium.

Stroma moderate phibrosis with ectatic and winding vessels; interstitial oedema with diffused neovascularisation presence of a severe and diffused inflammatory infiltrate.

The histological data are surprisingly very similar to those observed in control rabbits.

### Conclusions

The cornea histological tests highlighted not significant differences between the eyes instilled with the Ascotodin® eye drops and the eyes treated with physiological solution, in fact both treatments proved ineffective against UV damages. These data show that the UV screening substance contained in the Ascotodin® composition is not able to protect from UV damages when the contact between hydrogel vehicle and corneal mucosa is not long-lasting. Therefore carboxymethylcellulose in 0.033% concentration is not able to form a stable mucoadhesive film.

On the other hand, the results obtained with the Bruviscreen inventive formulation unexpectedly revealed that MBQM in the same concentration (0.3%) is very effective in protecting the corneal tissues from UV damages when formulated in the suitable long-term mucoadhesive hydrogel of the invention.

Actually the histological data of the tests performed on the cornea of the rabbits eyes instilled with Bruviscreen formulation highlighted significant differences versus the cornea tissues of eyes rabbits treated with Ascotodin® and with physiological solution.

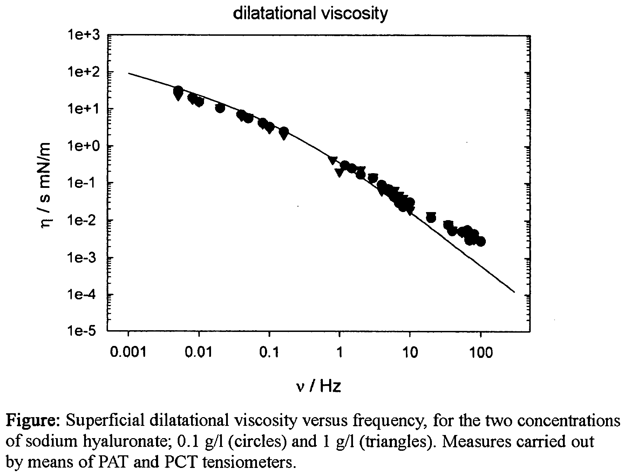

## Claims

1. An ophthalmic composition comprising:
a) n-methylbenzoquinoline methyl sulphate (MBQM) or its salts;
b) polyvinyl alcohol;
c) sodium hyaluronate; and
d) glycerin;
for the protection of the ocular tissues from the damaging effects of ultraviolet radiations.

2. The composition of claim 1, wherein n-methylbenzoquinoleine methyl sulphate having heavy metals content lower than 10 ppm is employed.

3. The composition of claims 1 and 2, wherein the various components are present in amounts within the following percentages by weight:
a) MBQM or its salts: 0.1% to 1%;
b) polyvinyl alcohol: 0.8% to 1.6%;
c) sodium hyaluronate: 0.005% to 0.1%; and
d) glycerin: 0.3 to 1.5%.

4. The composition of claim 3, wherein the various components are present in amounts within the following percentages by weight:
a) MBQM or its salts: 0.2% to 0.4%
b) polyvinyl alcohol: 1.0 to 1.4%;
c) sodium hyaluronate: 0.005% to 0.0020%; and
d) glycerin: 0.5% to 1.00%.

5. The composition of the preceding claims, comprising a pH buffer based on monobasic and dibasic sodium and/or potassium phosphates; boric acid /sodium tetraborate in combination with sodium/potassium bicarbonate or system citric acid /citrates, in amounts effective to maintain pH of said composition in a range of from 6.0-7.0, preferably from 6.4 to 6.8.

6. The composition of the preceding claims, further comprising conventional additives and osmolarity agents.

7. The composition of claim 6, wherein the osmolarity agent is sodium chloride in amounts ranging from 0.1% to 1.0%.

8. The composition of the preceding claims, further comprising conventional preservatives, selected from the group consisting of p-hydroxybenzoic acid esters, hexamethylene biguanide, benzyl and phenylethyl alcohols, and combinations thereof.

9. The composition of the preceding claims, further comprising disodium edetate in amounts ranging from 0.010% to 0.100% by weight of the composition.

10. The composition of the preceding claims, further comprising Hamamelis distilled water.

## Patentansprüche

1. Ophthalmische Zusammensetzung, umfassend:
a) n-Methylbenzochinolinmethylsulfat (MBQM) oder seine Salze;
b) Polyvinylalkohol;
c) Natriumhyaluronat; und
d) Glycerin;
zum Schutz der Augengewebe vor den schädlichen Wirkungen von ultravioletter Strahlung.

2. Zusammensetzung gemäß Anspruch 1, wobei n-Methylbenzochinolinmethylsulfat mit einem Schwermetallgehalt von unter 10 ppm eingesetzt wird.

3. Zusammensetzung gemäß Anspruch 1 und 2, wobei die verschiedenen Komponenten in Mengen innerhalb der folgenden Gewichtsprozentanteile vorhanden sind:
a) MBQM oder seine Salze: 0,1 bis 1 Gew.-%;
b) Polyvinylalkohol: 0,8 bis 1,6 Gew.-%;
c) Natriumhyaluronat: 0,005 bis 0,1 Gew.-%; und
d) Glycerin: 0,3 bis 1,5 Gew.-%.

4. Zusammensetzung gemäß Anspruch 3, wobei die verschiedenen Komponenten in Mengen innerhalb der folgenden Gewichtsprozentanteile vorhanden sind:
a) MBQM oder seine Salze: 0,2 bis 0,4 Gew.-%;
b) Polyvinylalkohol: 1,0 bis 1,4 Gew.-%;
c) Natriumhyaluronat: 0,005 bis 0,0020 Gew.-%; und
d) Glycerin: 0,5 bis 1,00 Gew.-%.

5. Zusammensetzung gemäß den vorstehenden Ansprüchen, umfassend einen pH-Puffer auf der Basis von einbasigen und zweibasigen Natrium- und/oder Kaliumphosphaten; Borsäure/Natriumtetraborat in Kombination mit Natrium-/Kaliumhydrogencarbonat oder dem System Zitronensäure/Citrate in Mengen, die bewirken, dass der pH-Wert der Zusammensetzung in einem Bereich von 6,0 bis 7,0, vorzugsweise 6,4 bis 6,8, gehalten wird.

6. Zusammensetzung gemäß den vorstehenden Ansprüchen, weiterhin umfassend herkömmliche Additive und Osmolaritätsmittel.

7. Zusammensetzung gemäß Anspruch 6, wobei es sich bei dem Osmolaritätsmittel um Natriumchlorid in Mengen im Bereich von 0,1% bis 1,0% handelt.

8. Zusammensetzung gemäß den vorstehenden Ansprüchen, weiterhin umfassend herkömmliche Konservierungsstoffe, die aus der Gruppe ausgewählt sind, die aus p-Hydroxybenzoesäureestern, Hexamethylenbiguanid, Benzyl- und Phenylethylalkohole und Kombinationen davon besteht.

9. Zusammensetzung gemäß den vorstehenden Ansprüchen, weiterhin umfassend Dinatriumedetat in Mengen im Bereich von 0,010 bis 0,100 Gew.-% der Zusammensetzung.

10. Zusammensetzung gemäß den vorstehenden Ansprüchen, weiterhin umfassend Hamamelis/destilliertes Wasser.

## Revendications

1. Composition ophtalmique comprenant :
a) du méthylsulfate de n-méthylbenzoquinoléine (MBQM) ou ses sels ;
b) de l'alcool polyvinylique ;
c) du hyaluronate de sodium ; et
d) de la glycérine ;
pour la protection des tissus oculaires contre les effets néfastes des rayonnements ultraviolets.

2. Composition selon la revendication 1, dans laquelle un méthylsulfate de n-méthylbenzoquinoléine ayant une teneur en métaux lourds inférieure à 10 ppm est utilisé.

3. Composition selon les revendications 1 et 2, dans laquelle les différents constituants sont présents en quantités dans les pourcentages en poids suivants :
a) le MBQM ou ses sels : 0,1 % à 1 % ;
b) l'alcool polyvinylique : 0,8 % à 1,6 % ;
c) le hyaluronate de sodium : 0,005 % à 0,1 % ; et
d) la glycérine : 0,3 à 1,5 %.

4. Composition selon la revendication 3, dans laquelle les différents constituants sont présents en quantités dans les pourcentages en poids suivants :
a) le MBQM ou ses sels : 0,2 % à 0,4 % ;
b) l'alcool polyvinylique : 1,0 à 1,4 % ;
c) le hyaluronate de sodium : 0,005 % à 0,0020 % ; et
d) la glycérine : 0,5 % à 1,00 %.

5. Composition selon les revendications précédentes, comprenant un tampon pH à base de phosphates de sodium et/ou de potassium monobasiques et dibasiques ; de l'acide borique/tétraborate de sodium en combinaison avec du bicarbonate de sodium/potassium ou un système d'acide citrique/citrates, en quantités efficaces pour maintenir le pH de ladite composition dans une plage de 6,0 à 7,0, de préférence de 6,4 à 6,8.

6. Composition selon les revendications précédentes, comprenant en outre des additifs classiques et des agents d'osmolarité.

7. Composition selon la revendication 6, dans laquelle l'agent d'osmolarité est le chlorure de sodium en des quantités allant de 0,1 % à 1,0 %.

8. Composition selon les revendications précédentes, comprenant en outre des conservateurs classiques, choisis dans le groupe constitué par les esters d'acide p-hydroxybenzoïque, l'hexaméthylène biguanide, les alcools benzylique et phényléthylique et les combinaisons de ceux-ci.

9. Composition selon les revendications précédentes, comprenant en outre de l'édétate disodique en des quantités allant de 0,010 % à 0,100 % en poids de la composition.

10. Composition selon les revendications précédentes, comprenant en outre de l'eau distillée d'Hamamelis.
